# EUROPEAN PATENT APPLICATION

(11) **EP 0 872 236 A1**
(43) Date of publication of application: **21.10.1998**
(21) Application number: 96935461.2
(22) Date of filing: 28.10.1996
(51) Int. Cl.: A61K 31/47, C07D 401/04

(54) **EYE DROPS**

(30) Priority: 27.10.1995 JP 280759/95
(71) Applicant: Schering-Plough Kabushiki Kaisha, Osaka-shi, Osaka 541 (JP)
(72) Inventor: FUJIMOTO, Takashi, Kurita-gun, Shiga 520-30 (JP); OKAZAKI, Kimiya, Yokkaichi-shi, Mie 510 (JP); KAMI, Hiroshi, Otsu-shi, Shiga 520-22 (JP); KASE, Koichiro, Otsu-shi, Shiga 520 (JP); YOSHIZAWA, Eiko, Koga-gun, Shiga 520-33 (JP); SHIBAHARA, Takeshi, Kurita-gun, Shiga 520-30 (JP)
(74) Representative: Isenbruck, Günter, Dr.
(86) International application number: JP9603139
(87) International publication number: WO9715307

(57) **Abstract**

Eye drops containing compounds represented by general formula (I), wherein X represents halogeno or hydrogen; and Y represents -COOR₁ or -SO₂R₂ (wherein R₁ and R₂ represent each alkyl, cycloalkyl, aryl, etc.).

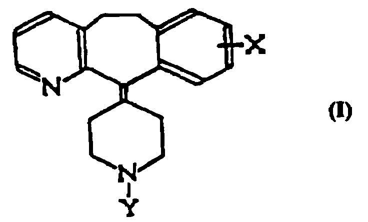

## Description

### Field of the Invention

The present invention relates to ophthalmic solutions containing a compound represented by formula (I) as an active ingredient.

### Prior Arts

It is known that some of the compounds represented by formula (I) are useful as non-sedative antihistaminics and act as antiallergics in the treatment of long-standing or climatic allergic rhinitis, chronic urticaria, etc. as reported in Japanese Patent Publication No. 55513/88. However, the study so far conducted on the antihistaminic action of the compound of formula (I) has been confined to oral administration, and the action exerted in other administration routes has not been given due study. In particular, even no suggestion has been made as to liquid preparations for application to the eye, and there is no knowledge available regarding the efficacy or proper composition as an ophthalmic solution.

It is an object of the present invention to provide an ophthalmic solution containing the compound of formula (I) as an active ingredient. An active ingredient must be present in a composition in at least an effective concentration before the composition can be used as an ophthalmic solution. However, since the compound of formula (I) is extremely sparingly soluble in water as having a water solubility as low as 3 ppm, it fails to provide a composition having an effective concentration when combined with a general solvent.

Further, an ophthalmic solution should be of low irritation and does not cause discomfort on use. Because the eye is especially sensitive to irritation, the composition of an ophthalmic solution should be studied carefully so as to be far less irritative than other preparation forms. It is also required of an ophthalmic solution to be highly stable and preservable for a relatively long period of time.

Thus, there are many problems to be solved before formulating ophthalmic solutions, and study should be given to combinations of various conditions and addition of new factors. However, there has been not a single report as would offer useful suggestions as to these subjects, still less application of the compound of formula (I) to an ophthalmic solution.

### Disclosure of the Invention

The present invention provides for the first time an ophthalmic solution containing the compound of formula (I).

The present invention discloses an ophthalmic solution containing a compound represented by formula (I): 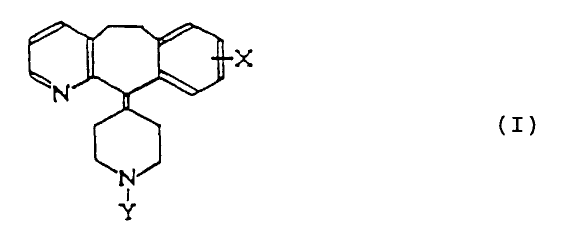 wherein X represents a halogen atom or a hydrogen atom; and Y represents -COOR₁ or -SO₂R₂, wherein R₁ represents a substituted or unsubstituted alkyl group, a substituted or unsubstituted cycloalkyl group, a substituted or unsubstituted alkenyl group, a substituted or unsubstituted aryl group, or a substituted or unsubstituted heterocyclic ring; and R₂ represents a substituted or unsubstituted alkyl group, a substituted or unsubstituted cycloalkyl group, or a substituted or unsubstituted aryl group.

The compounds of formula (I) embrace optical isomers (i.e., d-form and l-form) and mixtures thereof. The compounds of formula (I) can be prepared in accordance with processes known in the art, for example, the processes disclosed in Japanese Patent Publication No. 55513/88, U.S. Patent 3,326,924, and Belgian Patent 647,043.

Of the compounds represented by formula (I), preferred for application as ophthalmic solution are those in which X is a halogen atom or a hydrogen atom, and Y is -COOR, or -SO₂R₂, wherein R₁ represents an alkyl group having 1 to 12 carbon atoms which may be substituted with -NR₃R₄ (wherein R₃ and R₄ each represents a hydrogen atom or a lower alkyl group) or a halogen atom, a cycloalkyl group having 3 to 7 carbon atoms, a cycloalkylalkyl group having 4 to 12 carbon atoms, an alkenyl group having 3 to 12 carbon atoms, a phenyl group which may be substituted with a halogen atom or a lower alkyl group, a phenyl-lower alkyl group which has 1 to 4 carbon atoms in the alkyl moiety thereof and may be substituted with a halogen atom or a lower alkyl group at the phenyl moiety thereof, or a 2-, 3- or 4-piperidyl group which may be substituted with a lower alkyl group at the nitrogen atom thereof; and R₂ represents an alkyl group having 1 to 12 carbon atoms, a cycloalkyl group having 3 to 7 carbon atoms, a phenyl group which may be substituted with a halogen atom or a lower alkyl group, or a phenyl-lower alkyl group which has 1 to 4 carbon atoms in the alkyl moiety thereof and may be substituted with a halogen atom or a lower alkyl group at the phenyl moiety thereof.

Those in which X is a halogen atom or a hydrogen atom, and Y is a -COO-alkyl group are still preferred, with a compound wherein X is a chlorine atom at the 8-position, and Y is COOC₂H₅, called loratadine, and those equal or superior to loratadine in activity being particularly preferred.

The compound represented by formula (I) is used in an ophthalmic solution in an amount producing effective antihistaminic activity, which is usually selected from the range 0.01 to 100 mg/ml.

The ophthalmic solution of the present invention may contain solubilizers, stabilizers, preservatives, isotonic agents, and pH adjusting agents. Useful solubilizers include Polysorbate, propylene glycol, polyvinylpyrrolidone K30, and Poloxamer 188. Among them Polysorbate 80 is preferred for its excellent dissolving power and capability of maintaining an ophthalmic solution at an appropriate osmotic pressure as hereinafter verified in Test Example 3. Useful stabilizers include sodium hydrogensulfite, glycerol, sodium edetate, sodium citrate, and butylated hydroxyanisole. Among them sodium edetate is preferred for its excellent inhibitory action on coloration and capability of stably maintaining a prescribed pH and maintaining the high activity of the active compound as hereinafter proved in Test Example 4. The higher the concentration of benzalkonium chloride, the more stable the pH and activity of an ophthalmic solution. Therefore, benzalkonium chloride is preferably incorporated as a preservative in the maximum concentration described in the positive list of drug additives (0.127%). Useful isotonic agents include sodium chloride, D-mannitol, glucose, and glycerol. D-Mannitol is preferred for its excellent inhibitory action on coloration and capability of stably maintaining a prescribed pH and maintaining the high activity of the active compound as hereinafter proved in Test Example 5. Not observing concentration dependence of these activities of D-mannitol, D-mannitol is preferably used at such a concentration that makes an ophthalmic solution isotonic to physiological saline (1.53%). Useful pH adjusting agents include phosphate compounds, such as monobasic sodium phosphate (sodium dihydrogenphosphate) and dibasic sodium phosphate (disodium hydrogenphosphate), sodium hydroxide, and hydrochloric acid. In order to alleviate irritation on administration, the phosphate concentration is preferably not higher than 0.01 M, and it is recommended to use pH adjusting agents other than phosphate compounds, if possible, as in Test Example 8 hereinafter described. While the ophthalmic solution may have a pH of about 4 to 8, the pH is desirably brought to about 4 to 5 in order to maintain the stability of the active compound.

The ophthalmic solution according to the present invention exhibits excellent antiallergic activity and are effective on syndesmitis, hay fever, vernal conjunctivitis, and the like. In particular, the loratadine preparations disclosed in the present invention were verified to be about 40 times as active as ELICS™ or INTAL™ conventionally used as antiallergic ophthalmic solution, as hereinafter demonstrated in Test Example 1. In addition, there is no noticeable disturbances on the cornea, the iris or the conjunctiva upon instilling in the eye, proving high safety of the preparations of the present invention, as tested in Test Example 2.

The ophthalmic solution used in the present invention is preferably stored and used in a polypropylene container. Storage in polypropylene containers provides an excellent stability and accordingly use of polypropylene containers is advantageous compared with containers made of other materials.

While not limiting, the ophthalmic solution according to the present invention is typically administered at a dose of 1 to several drops 1 to 4 times a day according to the conditions.

### Examples

The present invention will now be illustrated in greater detail with reference to Examples.

### Examples 1 to 3

In distilled water was dissolved 23 g of Polysorbate 80 to prepare an about 300 ml solution. In the solution were successively dissolved 0.5 g of loratadine, 1.0 ml of 10% benzalkonium chloride, 1.27 g of sodium edetate, and 15.3 g of D-mannitol. To the solution was added 600 ml of distilled water, and the solution was subjected to any one of the following steps (i) to (iii) to prepare ophthalmic solution 1 to 3.
(i) To the solution was added 15.6 g of monobasic sodium phosphate, and the pH was adjusted to 5.0 with 0.1 M dibasic sodium phosphate. Distilled water was added thereto to make 1000 ml to prepare ophthalmic solution 1.
(ii) To the solution was added 1.56 g of monobasic sodium phosphate, and the pH was adjusted to 5.0 with 0.1 M dibasic sodium phosphate. Distilled water was added thereto to make 1000 ml to prepare ophthalmic solution 2.
(iii) The solution was adjusted to a pH of 5.0 with hydrochloric acid and a 1N sodium hydroxide aqueous solution, and distilled water was added thereto to make 1000 ml to prepare ophthalmic solution 3.

### Example 4

In distilled water was dissolved 23 g of Polysorbate 80 to prepare an about 300 ml solution. In the solution were successively dissolved 0.5 g of loratadine, 1.0 ml of 10% benzalkonium chloride, 1.27 g of sodium edetate, and 2.6 g of sodium chloride. To the solution was added 600 ml of distilled water, and the pH was adjusted to 5.0 with monobasic sodium phosphate and 0.1 M dibasic sodium phosphate. Distilled water was further added thereto to make 1000 ml to prepare ophthalmic solution 4.

### Example 5

In distilled water was dissolved 46 g of Polysorbate 80 to prepare an about 300 ml solution. In the solution were successively dissolved 1.0 g of loratadine, 1.0 ml of 10% benzalkonium chloride, 1.27 g of sodium edetate, and 2.6 g of sodium chloride. To the solution was added 600 ml of distilled water, and the pH was adjusted to 5.0 with monobasic sodium phosphate and 0.1 M dibasic sodium phosphate. Distilled water was further added thereto to make 1000 ml to prepare ophthalmic solution 5.

### Test Example 1

Anti-inflammatory activity on syndesmitis of ophthalmic solutions 1, 4 and 5 prepared in the foregoing Examples, a placebo of ophthalmic solution 1, ELICS™ (active ingredient: amlexanox), and INTAL™ (active ingredient: cromoglicic acid) was examined as follows.

An anti-egg albumin serum of a guinea pig was prepared as follows. Guinea pigs weighing 200 to 300 g were sensitized by intraperitoneal administration of 1 ml of a 1 mg/ml solution of egg albumin in physiological saline and 1 ml of pertussis-diphtheria-tetanus vaccine (2 x 10¹⁰ cells/ml). Four weeks later, the animals were decapitated and the blood collected. After being allowed to stand at room temperature for 30 minutes and 4°C or lower for 4 to 5 hours, the blood was centrifuged at 3000 rpm for 15 minutes to obtain antiserum.

The above prepared guinea pig anti-egg albumin serum (antibody titer in PCA reaction: 1.32) (0.05 ml) was intracutaneously administered to the conjunctiva of the superior eyelid of Hartley male guinea pigs weighing 300 to 500 g to conduct passive sensitization. After 7 days from the sensitization, 1 ml of a 1:1 mixture of 1% egg albumin and 1% Evans' Blue was injected to the vein of the back of the hind limb to induce allogenic PCA at the conjunctiva in order to observe the response to the allogenic IgE antibody. After 30 minutes from the reaction induction, the animals were decapitated and the conjunctive tissue where the dye was extravasated was dissected and minced. The dye was extracted with a 7:3 mixture of acetone and 0.5% sodium sulfate overnight, and the amount of the extravasated dye was measured with a spectrophotometer (wavelength: 625 nm). One drop of the ophthalmic solution was instilled in both eyes of the animals 3, 2, 1 and 0.5 hour before the induction of PCA reaction and immediately after the PCA induction.

The results obtained are shown in Table 1 below. In the Table, amount of extravasated dye is expressed in terms of mean (n=8) ± standard error, and percent inhibition was calculated based on the result of the physiological saline group.

**Table 1**

| Ophthalmic Solution | Concn. of Active Ingredient (%) | Amount of Extravasated Dye (µg/site) | Percent Inhibition (%) |
|---|---|---|---|
| Physiological saline | 0 | 10.0±0.7 | - |
| Ophthalmic solution 1 | 0.05 | 6.8±1.0 | 37.5 |
| Ophthalmic solution 4 | 0.05 | 6.3±1.2 | 42.1 |
| Ophthalmic solution 5 | 0.1 | 6.3±0.8 | 42.3 |
| Placebo | 0 | 10.2±1.0 | 7.0 |
| ELICS | 0.25 | 11.2±1.7 | -1.3 |
| INTAL | 2.0 | 1.2±2.1 | -1.4 |

The anti-inflammatory activity of ophthalmic solution 2, a placebo thereof, ZADITEN™ (active ingredient: Ketotifen fumarate), and INTAL™ was examined in the same manner as described above. The results obtained are shown in Table 2 below.

**Table 2**

| Ophthalmic Solution | Dose (%) | Amount of Extravasated Dye (µg/site) | Percent Inhibition (%) |
|---|---|---|---|
| Physiological saline | 0 | 15.7±0.8 | - |
| Ophthalmic solution 2 | 5 | 11.1±1.3 | 29.6 |
| Placebo | 0 | 16.3±1.3 | -3.4 |
| ZADITEN | 5 | 9.7±1.0 | 38.3 |
| INTAL | 200 | 12.9±1.1 | 17.9 |

### Test Example 2

An irritation test on the ophthalmic mucosa was carried out using ophthalmic solutions 1, 4 and 5 prepared in the foregoing Examples.

Four Japanese white rabbits with no abnormalities found in their eyes were used per group for each sample. Each sample and physiological saline were instilled in the right eye and the left eye, respectively, at a dose of 2 drops every 30 minutes. The eyes were observed 1 hour, 1, 2, 5 and 7 days after the instillation and judged according to Draize' method. As a result, no disturbances of the cornea, the iris or the conjunctiva was observed for every sample, verifying that short-term instillation involves no problematical irritation to the eyes.

### Test Example 3

A solubilization test was carried out using various solubilizers.

A prescribed amount of the solubilizer shown in Table 3 below was dissolved in distilled water to prepare an about 300 ml solution. To the solution were successively added 0.5 g of loratadine and 1.0 ml of 10% benzalkonium chloride, followed by stirring. To the mixture was further added 600 ml of distilled water, and 5.6 g of monobasic sodium phosphate and 2.8 g of dibasic sodium phosphate were then added thereto. Finally, distilled water was added to make 1000 ml, and the condition of the mixture was observed (pH 6.3-6.6).

**Table 3**

| Sample No. | Solubilizer |
|---|---|
| 1 | Polysorbate 80 (23 g) |
| 2 | Polysorbate 80 (4 g) + propylene glycol (50 g) |
| 3 | Polysorbate 80 (23 g) + propylene glycol (50 g) |
| 4 | Polyvinylpyrrolidone K30 (3 g) + propylene glycol (50 g) |
| 5 | Polyvinylpyrrolidone K30 (10 g) |
| 6 | Polyvinylpyrrolidone K30 (50 g) |
| 7 | Polyvinylpyrrolidone K30 (100 g) |
| 8 | Poloxamer 188 (Pluronic™ F68) (50 g) |
| 9 | Poloxamer 188 (Pluronic™ F68) (200 g) |
| 10 | Poloxamer 235 (Pluronic™ P85) (50 g) |
| 11 | Poloxamer 235 (Pluronic™ P85) (200 g) |

The results of the observation are shown in Table 4 below.

**Table 4**

| Sample No. | Condition of Loratadine | Osmotic Pressure* |
|---|---|---|
| 1 | dissolved | 0.54 |
| 2 | suspended | 2.92 |
| 3 | dissolved | 3.02 |
| 4 | suspended | 2.89 |
| 5 | suspended | 0.51 |
| 6 | suspended | 0.60 |
| 7 | suspended | 0.78 |
| 8 | suspended | 0.68 |
| 9 | suspended | ≥3.5 |
| 10 | suspended | 0.72 |
| 11 | dissolved | ≥3.5 |

| | | |
|---|---|---|
| Note: * The osmotic pressure of physiological saline was taken as 1. | | |

### Test Example 4

A stabilization test was conducted using various stabilizers.

In distilled water was dissolved 23 g of Polysorbate 80 to prepare an about 300 ml solution. To the solution were successively added 0.5 g of loratadine and the stabilizer shown in Table 5 below in the concentration shown (maximum concentration described in the positive list of drug additives), followed by stirring. Distilled water was added thereto to make 1000 ml, and the resulting solution was preserved at 60°C. APHA No., pH, and loratadine retention (measured by HPLC) were examined at the start of the preservation and after 1, 2, and 5 weeks' preservation.

**Table 5**

| Sample No. | Stabilizer (mg/ml) |
|---|---|
| 1 | Sodium hydrogensulfite (5 g) |
| 2 | Glycerol (200 g) |
| 3 | 10% Benzalkonium chloride (1.0 ml) |
| 4 | Sodium edetate (1.27 g) |
| 5 | Sodium citrate (20 g) |
| 6 | Butylated hydroxyanisole (2 g) |
| 7 | None |
| 8 | None* |

| | |
|---|---|
| Note: * Containing no loratadine. | |

The results obtained are shown in Table 6 below.

**Table 6**

| Sample No. | Testing Item | Initial | 1 Week | 2 Weeks | 5 Weeks |
|---|---|---|---|---|---|
| 1 | APHA No. | 29 | 35 | 39 | 91 |
| | pH | 3.62 | 2.74 | 2.69 | 2.62 |
| | Loratadine retention (%) | 100 | 98.3 | 97.7 | 94.4 |
| 2 | APHA No. | 18 | 45 | 80 | 127 |
| | pH | 6.29 | 3.94 | 3.75 | 3.62 |
| | Loratadine retention (%) | 100 | 82.0 | 75.9 | 65.3 |
| 3 | APHA No. | 19 | 56 | 91 | 188 |
| | pH | 6.10 | 3.78 | 3.57 | 3.42 |
| | Loratadine retention (%) | 100 | 76.8 | 67.4 | 48.6 |
| 4 | APHA No. | 22 | 31 | 31 | 46 |
| | pH | 5.07 | 5.13 | 5.05 | 4.72 |
| | Loratadine retention (%) | 100 | 100.1 | 98.0 | 92.2 |
| 5 | APHA No. | 22 | 26 | 43 | 90 |
| | pH | 7.61 | 7.34 | 6.97 | 6.77 |
| | Loratadine retention (%) | 100 | 95.9 | 79.8 | 63.2 |
| 6 | APHA No. | 70 | 513 | 386 | 874 |
| | pH | 6.38 | 4.55 | 3.58 | 3.68 |
| | Loratadine retention (%) | 100 | 98.3 | 100.1 | 96.6 |
| 7 | APHA No. | 26 | 49 | 70 | 194 |
| | pH | 6.41 | 3.78 | 3.58 | 3.44 |
| | Loratadine retention (%) | 100 | 75.2 | 67.9 | 51.5 |
| 8 | APHA No. | 23 | 15 | 18 | 22 |
| | pH | 6.47 | 3.55 | 3.39 | 3.27 |
| | Loratadine retention (%) | - | - | - | - |

### Test Example 5

An examination was carried out using various isotonic agents.

In distilled water was dissolved 23 g of Polysorbate 80 to prepare an about 300 ml solution. In the solution were successively dissolved 0.5 g of loratadine, 1.0 ml of 10% benzalkonium chloride, and 1.27 g of sodium edetate. An isotonic agent shown below was added to the solution in the amount shown to make the solution isotonic to physiological saline.
- Sample 1:: Sodium chloride (2.6 g)
- Sample 2:: D-Mannitol (15.3 g)
- Sample 3:: Glucose (14.5 g)
- Sample 4: Glycerol (7.5 g)

To the solution was added 600 ml of distilled water, and the solution was adjusted to pH 5.0 with monobasic sodium phosphate and dibasic sodium phosphate. Distilled water was further added to make 1000 ml. Each sample was preserved at 40°C, and APHA No., pH, and loratadine retention were measured at the start of the preservation and after 2, 4 and 6 months' preservation in the same manner as in Test Example 3. A preservation test at 60°C was also carried out, and measurements were made after 1, 2, and 5 weeks' and 2 months' preservation. The results obtained are shown in Tables 7 and 8 below.

**Table 7**

| Preservation at 40°C | | | | | |
|---|---|---|---|---|---|
| Sample No. | Testing Item | Initial | 2 Mths. | 4 Mths. | 6 Mths. |
| 1 | APHA No. | 23 | 30 | 26 | 29 |
| | pH | 4.98 | 4.94 | 4.89 | 4.87 |
| | Loratadine retention (%) | 100 | 97.7 | 95.3 | 94.5 |
| 2 | APHA No. | 22 | 28 | 27 | 29 |
| | pH | 4.98 | 4.95 | 4.93 | 4.90 |
| | Loratadine retention (%) | 100 | 98.0 | 96.8 | 97.0 |
| 3 | APHA No. | 19 | 30 | 27 | 28 |
| | pH | 4.97 | 4.95 | 4.90 | 4.88 |
| | Loratadine retention (%) | 100 | 97.9 | 96.3 | 95.5 |
| 4 | APHA No. | 25 | 30 | 29 | 27 |
| | pH | 4.98 | 4.96 | 4.90 | 4.89 |
| | Loratadine retention (%) | 100 | 98.4 | 93.8 | 95.5 |

**Table 8**

| Preservation at 60°C | | | | | |
|---|---|---|---|---|---|
| Sample No. | Testing Item | 1 Wk. | 2 Wks. | 5 Wks. | 2 Mths. |
| 1 | APHA No. | 35 | 27 | 49 | 71 |
| | pH | 4.91 | 4.93 | 4.80 | 4.31 |
| | Loratadine retention (%) | 98.7 | 94.5 | 87.1 | 68.7 |
| 2 | APHA No. | 31 | 31 | 40 | 56 |
| | pH | 4.92 | 4.94 | 4.88 | 4.60 |
| | Loratadine retention (%) | 98.2 | 97.1 | 94.2 | 80.5 |
| 3 | APHA No. | 40 | 31 | 46 | 59 |
| | pH | 4.92 | 4.92 | 4.80 | 4.50 |
| | Loratadine retention (%) | 98.6 | 97.5 | 91.0 | 78.5 |
| 4 | APHA No. | 35 | 26 | 46 | 58 |
| | pH | 4.91 | 4.92 | 4.81 | 4.44 |
| | Loratadine retention (%) | 97.8 | 96.6 | 90.7 | 74.3 |

### Test Example 6

A stabilization test was applied to ophthalmic solutions having a varied pH.

In distilled water was dissolved 23 g of Polysorbate 80 to prepare an about 300 ml solution. In the solution were successively dissolved 0.5 g of loratadine, 1.0 ml of 10% benzalkonium chloride, 1.27 g of sodium edetate, and 2.6 g of sodium chloride. To the solution was added 600 ml of distilled water, and the pH was adjusted to 4, 5, 6, 7 or 8 with monobasic sodium phosphate and dibasic sodium phosphate. Distilled water was further added thereto to make 1000 ml. The resulting sample was preserved at 40°C, and APHA No., pH, and loratadine retention were measured at the start of the preservation and after 2, 4 and 6 months' preservation in the same manner as in Test Example 3. A preservation test at 60°C was also carried out, and measurements were made after 1, 2, and 5 weeks' preservation. The results obtained are shown in Tables 9 and 10.

**Table 9**

| Preservation at 40°C | | | | | |
|---|---|---|---|---|---|
| pH | Testing Item | Initial | 2 Mths. | 4 Mths. | 6 Mths. |
| 4 | APHA No. | 26 | 29 | 19 | 27 |
| | pH | 4.04 | 4.04 | 4.04 | 3.98 |
| | Loratadine retention (%) | 100 | 99.1 | 99.0 | 95.1 |
| 5 | APHA No. | 25 | 40 | 17 | 28 |
| | pH | 5.03 | 5.00 | 4.98 | 4.95 |
| | Loratadine retention (%) | 100 | 98.8 | 98.1 | 94.2 |
| 6 | APHA No. | 25 | 38 | 28 | 46 |
| | pH | 6.03 | 5.99 | 5.98 | 5.92 |
| | Loratadine retention (%) | 100 | 96.5 | 85.2 | 64.6 |
| 7 | APHA No. | 25 | 40 | 62 | 80 |
| | pH | 7.00 | 6.98 | 6.95 | 6.92 |
| | Loratadine retention (%) | 100 | 80.8 | 67.4 | 56.3 |
| 8 | APHA No. | 24 | 50 | 54 | 82 |
| | pH | 7.98 | 7.82 | 7.68 | 7.57 |
| | Loratadine retention (%) | 100 | 79.7 | 64.6 | 54.8 |

**Table 10**

| Preservation at 60°C | | | | |
|---|---|---|---|---|
| pH | Testing Item | 1 Wk. | 2 Wks. | 5 Wks. |
| 4 | APHA No. | 58 | 40 | 44 |
| | pH | 4.00 | 4.04 | 3.96 |
| | Loratadine retention (%) | 99.0 | 97.2 | 91.3 |
| 5 | APHA No. | 49 | 41 | 48 |
| | pH | 4.97 | 5.02 | 4.78 |
| | Loratadine retention (%) | 98.8 | 97.8 | 87.1 |
| 6 | APHA No. | 56 | 40 | 100 |
| | pH | 5.97 | 6.02 | 5.87 |
| | Loratadine retention (%) | 97.2 | 90.5 | 59.7 |
| 7 | APHA No. | 63 | 72 | 136 |
| | pH | 6.95 | 6.98 | 6.95 |
| | Loratadine retention (%) | 92.2 | 76.6 | 63.5 |
| 8 | APHA No. | 46 | 54 | 171 |
| | pH | 7.87 | 7.77 | 7.56 |
| | Loratadine retention (%) | 96.3 | 83.1 | 63.7 |

### Test Example 7

Ophthalmic solutions having a varied pH were prepared in the same manner as in Test Example 6 except for replacing 2.6 g of sodium chloride with 15.3 g of D-mannitol, and the same tests as in Test Example 6 were applied. In the stabilization test at 40°C, measurements were made at 2 months' preservation, and in the test at 60°C, at 1, 2 and 5 weeks' and 2 months' preservation. The results obtained are shown in Tables 11 and 12 below.

**Table 11**

| Preservation at 40°C | | | |
|---|---|---|---|
| pH | Testing Item | Initial | 2 Mths. |
| 4 | APHA No. | 29 | 26 |
| | pH | 4.00 | 3.95 |
| | Loratadine retention (%) | 100 | 99.1 |
| 5 | APHA No. | 25 | 25 |
| | pH | 5.01 | 4.99 |
| | Loratadine retention (%) | 100 | 98.8 |
| 6 | APHA No. | 24 | 23 |
| | pH | 5.99 | 5.99 |
| | Loratadine retention (%) | 100 | 96.9 |
| 7 | APHA No. | 27 | 24 |
| | pH | 6.97 | 6.95 |
| | Loratadine retention (%) | 100 | 89.1 |
| 8 | APHA No. | 27 | 27 |
| | pH | 7.95 | 7.80 |
| | Loratadine retention (%) | 100 | 89.7 |

**Table 12**

| Preservation at 60°C | | | | | |
|---|---|---|---|---|---|
| pH | Testing Item | 1 Wk. | 2 wks. | 5 Wks. | 2 Mths. |
| 4 | APHA No. | 29 | 27 | 33 | 46 |
| | pH | 4.03 | 3.97 | 3.87 | 3.84 |
| | Loratadine retention (%) | 100.5 | 98.1 | 93.0 | 86.1 |
| 5 | APHA No. | 25 | 25 | 33 | 49 |
| | pH | 5.03 | 5.05 | 4.88 | 4.67 |
| | Loratadine retention (%) | 99.6 | 98.0 | 91.0 | 80.9 |
| 6 | APHA No. | 30 | 28 | 58 | 110 |
| | pH | 6.01 | 6.02 | 5.89 | 5.76 |
| | Loratadine retention (%) | 97.8 | 96.2 | 68.8 | 50.0 |
| 7 | APHA No. | 30 | 33 | 106 | 197 |
| | pH | 7.00 | 6.97 | 6.87 | 6.81 |
| | Loratadine retention (%) | 97.0 | 86.8 | 60.4 | 51.7 |
| 8 | APHA No. | 28 | 30 | 116 | 211 |
| | pH | 7.93 | 9.82 | 7.49 | 7.36 |
| | Loratadine retention (%) | 101.3 | 92.4 | 64.9 | 57.2 |

### Test Example 8

Irritation of ophthalmic solutions to the eyes was examined.

Ophthalmic solutions having a phosphate buffer concentration of 0.1 M, 0.05 M, 0.02 M, 0.01 M, and 0.005 M were prepared in the same manner as in Example 1 (designated samples 1 to 5). Two drops of each of samples 1 to 5, ophthalmic solution 3 prepared in Example 3, INTAL™, and ZADITEN™ were instilled in one eye of subjects, and the ocular pain after 10 minutes from the instillation was evaluated according to the following rating system.
0 ... Painlessness
1 ... Very weak pain
2 ... Weak pain
3 ... Moderate pain
4 ... Strong pain
5 ... Very strong pain

An averaged score of 5 subjects was as shown in Table 13 below.

**Table 13**

| Ophthalmic Solution | Average Score | Evaluation |
|---|---|---|
| Sample 1 (0.1 M*) | 4 | Strong pain |
| Sample 2 (0.05 M*) | 3 | Moderate pain |
| Sample 3 (0.02 M*) | 2 | Weak pain |
| Sample 4 (0.01 M*) | 2 | Weak pain |
| Sample 5 (0.005 M*) | 2 | Weak pain |
| Ophthalmic solution 3 | 1 | Very weak pain |
| INTAL | 1 | Very weak pain |
| ZADITEN | 2 | Weak pain |

### Test Example 9

Stability during storage in various containers was tested.

The ophthalmic solutions 1 - 3 prepared by the manners described in Examples 1 - 3 were stored in a low-density polyethylene container (LDPE), a polyethyleneterephthalate container (PET), a polypropylene container (PP) and a 5 ml glass vial container with a butyl rubber stopper (VIAL). After 3 and 6 months storage at 25°C and after 1, 3 and 6 months storage at 40°C, the weights of the containers and the solutions were weighed and loratadine concentrations in the solutions were determined by HPLC. When LDPE was used, the weight of the container and the solution was not changed significantly but the maximum loss of loratadine concentration was 20.0%.
It is indicated that when LDEP was used as a container, loratadine was absorbed in LDPE. When PET was used, the maximum loss of the weight of the container and the solution was 15.2% and maximum increment of the loratadine concentration was 17.0%. It is indicated that when PET was used as a container, water was volatiled from the container during storage to concentrate loratadine. When VIAL was used, the maximum loss of the loratadine concentration was 7.6%. It is indicated that when VIAL was used as a container, loratadine was absorbed in the butyl rubber stopper. On the other hand, when PP was used as a container, no problems were observed in the change of the weight of the container and the solution and the loratadine concentration.

## Claims

1. An ophthalmic solution containing a compound represented by formula (I): 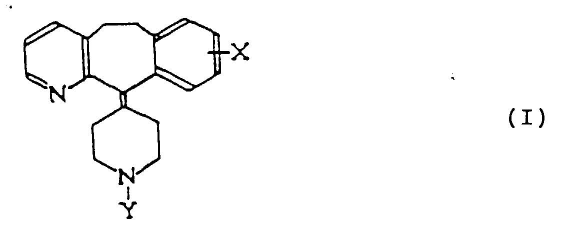 wherein X represents a halogen atom or a hydrogen atom; and Y represents -COOR₁ or -SO₂R₂, wherein R₁ represents a substituted or unsubstituted alkyl group a substituted or unsubstituted cycloalkyl group, a substituted or unsubstituted alkenyl group, a substituted or unsubstituted aryl group, or a substituted or unsubstituted heterocyclic ring; and R₂ represents a substituted or unsubstituted alkyl group, a substituted or unsubstituted cycloalkyl group, or a substituted or unsubstituted aryl group.

2. An ophthalmic solution containing a compound represented by formula (I): 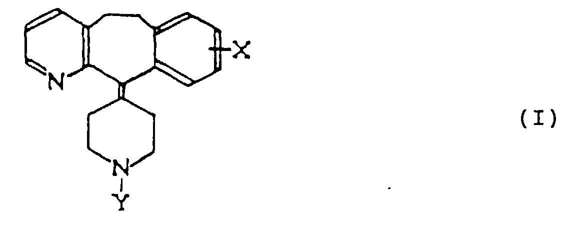 wherein X represents a halogen atom or a hydrogen atom; and Y represents -COOR₁ or -SO₂R₂, wherein R₁ represents an alkyl group having 1 to 12 carbon atoms which may be substituted with -NR₃R₄ (wherein R₃ and R₄ each represents a hydrogen atom or a lower alkyl group) or a halogen atom, a cycloalkyl group having 3 to 7 carbon atoms, a cycloalkylalkyl group having 4 to 12 carbon atoms, an alkenyl group having 3 to 12 carbon atoms, a phenyl group which may be substituted with a halogen atom or a lower alkyl group, a phenyl-lower alkyl group which has 1 to 4 carbon atoms in the alkyl moiety thereof and may be substituted with a halogen atom or a lower alkyl group at the phenyl moiety thereof, or a 2-, 3- or 4-piperidyl group which may be substituted with a lower alkyl group at the nitrogen atom thereof; and R₂ represents an alkyl group having 1 to 12 carbon atoms, a cycloalkyl group having 3 to 7 carbon atoms, a phenyl group which may be substituted with a halogen atom or a lower alkyl group, or a phenyl-lower alkyl group which has 1 to 4 carbon atoms in the alkyl moiety thereof and may be substituted with a halogen atom or a lower alkyl group at the phenyl moiety thereof.

3. Application of the compound described in claim 1 or 2 to an ophthalmic solution.

4. An ophthalmic solution according to claim 1 or 2, wherein said ophthalmic solution further contains as a solubilizer Polysorbate 80 or a compound equivalent or superior thereto.

5. An ophthalmic solution according to claim 1 or 2, wherein said ophthalmic solution further contains as a stabilizer sodium edetate or a compound equivalent or superior thereto.

6. An ophthalmic solution according to claim 1 or 2, wherein said ophthalmic solution further contains as a preservative benzalkonium chloride or a compound equivalent or superior thereto.

7. An ophthalmic solution according to claim 1 or 2, wherein said ophthalmic solution further contains as an isotonic agent D-mannitol or a compound equivalent or superior thereto.

8. An ophthalmic solution according to claim 1 or 2, wherein said ophthalmic solution has a phosphate concentration of not higher than 0.01 M.

9. An ophthalmic solution according to claim 1 or 2, wherein said ophthalmic solution has a pH of 4 to 5.

10. An ophthalmic solution according to claim 1 or 2, wherein said ophthalmic solution further contains Polysorbate 80 or a solubilizing agent equivalent or superior thereto, sodium edetate or a stabilizer equivalent or superior thereto, benzalkonium chloride or a preservative agent equivalent or superior thereto, and D-mannitol or an isotonic agent equivalent or superior thereto, and having a phosphate concentration of not higher than 0.01 M and a pH of 4 to 5.

11. An ophthalmic solution product which contains an ophthalmic solution according to any one of claims 1, 2 and 4 - 10 in a polypropylene container.
